# EUROPÄISCHE PATENTSCHRIFT

(11) **EP 1 287 795 B1**
(45) Veröffentlichungstag und Bekanntmachung des Hinweises auf die Patenterteilung: **18.06.2008**
(21) Anmeldenummer: 01810828.2
(22) Anmeldetag: 24.08.2001
(51) Int. Cl.: A61F 2/44

(54) **Künstliche Bandscheibe**
ARTIFICIAL SPINAL DISC
DISQUE INTERVERTEBRAL

(43) Veröffentlichungstag der Anmeldung: 05.03.2003
(73) Patentinhaber: Zimmer GmbH, 8404 Winterthur (CH)
(72) Erfinder: Casutt, Simon, 9200 Gossau (CH)
(74) Vertreter: Manitz, Finsterwald & Partner GbR

(56) Entgegenhaltungen:
- EP-A- 0 346 269
- DE-C- 4 315 757
- DE-U- 20 019 520
- FR-A- 2 787 016
- FR-A- 2 787 018

## Beschreibung

Die Erfindung betrifft eine künstliche Bandscheibe gemäss dem Oberbegriff des unabhängigen Anspruchs 1.

Die Bandscheibe übernimmt in der Wirbelsäule gleich mehrere zentrale Funktionen. Sie wirkt als Dämpfer, Abstandskörper und auch als Gelenk zwischen den Wirbelkörpern. Entsprechend vielschichtig sind die Anforderungen, die an ein Implantat zu stellen sind, das in seiner Funktion als künstliche Bandscheibe als Ersatz für eine natürliche Bandscheibe dienen soll. So muss die künstliche Bandscheibe selbstverständlich aus biokompatiblen Materialien aufgebaut sein und als Dauerimplantat für den Patienten möglichst lebenslang ihre Funktion erfüllen. Neben der einfachen Funktion als Abstandskörper muss die künstliche Bandscheibe insbesondere die in der Wirbelsäule auftretenden Stosskräfte wirksam abfedern können, damit die Wirbel nicht überlastet werden, ohne dabei jedoch die Wirbelbeweglichkeit merklich zu behindern. Um die natürlichen Dreh-, Kippund Schubbelastungen, wie sie in der Wirbelsäule typischerweise auftreten, geeignet ableiten zu können, muss eine feste Verbindung zwischen der künstlichen Bandscheibe und angrenzendem Wirbel gewährleistet sein.

Insbesondere an die elastischen Eigenschaften der künstlichen Bandscheibe, sowohl was deren Verhalten bzgl. Torsions-, Biege- und Schubbelastungen, als auch in Bezug auf Druckbelastungen angeht, sind somit höchste Anforderungen zu stellen. Insgesamt bedeutet das, dass die mechanischen Eigenschaften der künstlichen Bandscheibe möglichst identisch denjenigen der natürlichen Bandscheibe nachzubilden sind.

Zur Nachahmung der natürlichen Eigenschaften einer Bandscheibe sind zahlreiche Lösungsansätze bekannt. So sind künstliche Bandscheiben mit der EP-0 346 269 B1 bekannt, die aus zwei gegenüber angeordneten Endplatten bestehen, die über ein Wellrohr verbunden und mit einem viskoelestischen Material gefüllt sind. Das bisher ungelöste Problem solcher Anordnungen besteht jedoch unter anderem darin, die passende, d.h. die natürliche, stark nicht-lineare, Charakteristik einer Bandscheibe im üblichen Belastungsbereich für Druck-, Zug-, Schub-, Biege-, Flexions- und Torsionsbeanspruchungen bei einer künstlichen Bandscheibe nachzubilden.

Die Aufgabe der Erfindung ist es daher, eine künstliche Bandscheibe vorzuschlagen, welche die komplexen elastischen Eigenschaften einer natürlichen Bandscheibe möglichst exakt nachbildet.

Die diese Aufgabe lösende künstliche Bandscheibe ist durch die Merkmale des unabhängigen Anspruchs 1 gekennzeichnet. Die abhängigen Ansprüche beziehen sich auf besonders vorteilhafte Ausführungsformen der Erfindung.

Die erfindungsgemässe künstliche Bandscheibe zum Implantieren zwischen zwei benachbarte Wirbelkörper umfasst zwei Endplatten, die einen mit einem elastisch und / oder plastisch verformbaren Nucleus ausgefüllten Hohlraum an zwei gegenüberliegenden Seiten begrenzen. Dabei ist der Hohlraum von einem rohrförmigen Faserring derart umschlossen, dass die Endplatten zusammen mit dem Faserring den Hohlraum begrenzen und die Endplatten mit dem Faserring in zugfester Verbindung stehen. Der Faserring ist in radialer und axialer Richtung elastisch dehnbar ausgeführt, wobei der Faserring ein grösseres Elastizitätsmodul als der Nucleus aufweist. Der Nucleus und der Faserring wirken derart zusammen, dass die elastischen Eigenschaften der künstlichen Bandscheibe mindestens in Bezug auf eine Kompressionskraft mit zunehmender Verformung ein nicht-lineares Verhalten zeigen.

Bei der erfindungsgemässen künstlichen Bandscheibe sind zwei sich gegenüberstehende Endplatten an ihrer Peripherie mittels eines elastisch verformbaren Faserrings verbunden, welcher auf die Endplatten im unbelasteten Zustand eine ständige Zugkraft ausübt, so dass der Nucleus, der sich zwischen den beiden Endplatten innerhalb des Faserrings befindet, unter Druckvorspannung steht. Ein wesentlicher Nachteil bekannter künstlicher Bandscheiben besteht darin, dass unter der Wirkung einer elastischen Deformation entweder zuviel Weg beansprucht wird, bis der eigentliche Belastungsbereich erreicht ist, oder bereits die Anfangssteifigkeit zu hohe Werte aufweist. Ein entscheidender Vorteil der erfindungsgemässen künstlichen Bandscheibe ist daher darin zu sehen, dass deren elastisches Verhalten, zumindest in Bezug auf eine Kompression, bereits bei kleinen Deformationen ein nicht-lineares Verhalten zeigt.

Das elastische Verhalten der erfindungsgemässen künstlichen Bandscheibe ist dabei durch die den Faserring und den Nucleus umfassenden Materialien, sowie insbesondere durch die konstruktionsbedingte mechanische Kopplung von Faserring und den daran anliegenden Nucleus bestimmt. Dabei ist unter anderem der konkrete Aufbau des Faserrings von besonderer Bedeutung für die elastischen Eigenschaften der künstlichen Bandscheibe. So kann das mechanische Verhalten des Faserrings bezüglich einer Dehnung unter Druckbelastung, sowie einer Dehnung z.B. infolge von Torsions- Biege- oder Querverlagerungsbewegungen schon durch die Art des Gewebeaufbaus verschieden sein, je nachdem ob der Faserring gewebt, gestrickt, geflochten oder auf andere Weise hergestellt wurde. Dabei spielt unter anderem die Orientierung der Fasern, aus denen der Faserring aufgebaut ist, in ihrer Schräglage zur Richtung der Längsachse der künstlichen Bandscheibe eine Rolle für die zwischen den Endplatten zu übertragenden Kräfte. Durch die Kombination von Faserring und Nucleus und eine entgegengesetzte Schrägstellung der Fasern relativ zur Richtung der Längsachse der künstlichen Bandscheibe, kann eine Umsetzung der verschiedenen auftretenden Belastungen in eine Kompression der Scheibe erreicht werden.

Der Nucleus wird als Fluid durch eine verschliessbare Öffnung, die sich in einer der Deckplatten befindet, unter Druck in die künstliche Bandscheibe eingefüllt. Unter einem Fluid kann man im weitesten Sinne auch zum Beispiel ein in Körner- oder Pulverform vorliegendes getrocknetes Gel oder eine Substanz vergleichbarer Konsistenz verstehen. Dabei kann das Fluid nach einer gewissen Zeit zu einem elastischen oder zumindest teilweise plastischen Körper verfestigen oder seine fluiden Eigenschaften als mehr oder weniger zähe Flüssigkeit auf Dauer beibehalten. Entscheidend für die Funktion der künstlichen Bandscheibe ist, dass der so gebildete Nucleus einerseits den Hohlraum, den die Deckplatten mit dem Faserring bilden, bevorzugt vollständig ausfüllt und andererseits dauerhaft wenig inkrompressibel ist und unter Druckvorspannung steht. Dadurch ist gewährleistet, dass der Nucleus in unmittelbarer Wirkverbindung mit dem Faserring steht und so als Krafttransformator wirken kann. Wird die so aufgebaute künstliche Bandscheibe z.B. einer Druckbelastung ausgesetzt, so muss der Nucleus unter der Kompressionswirkung teilweise radial nach aussen ausweichen und dehnt dadurch den Faserring, wodurch die Kräfte aus der Druckbelastung zumindest teilweise in den Faserring transformiert werden.

Da der Nucleus bereits im unbelasteten Zustand über den Faserring unter einer gewissen Druckvorspannung, die durch den Fülldruck in weiten Grenzen beliebig eingestellt werden kann, steht, kann die Anfangssteifigkeit schon allein durch den Fülldruck des Fluids justiert werden. Damit besteht die Möglichkeit, unter Einhaltung einer vorgegebenen Geometrie für die künstliche Bandscheibe und ohne Veränderung oder Austausch der die künstliche Bandscheibe aufbauenden Materialien, die elastischen Eigenschaften an die individuellen Bedürfnisse, eines Patienten anzupassen.

So kann die nicht-lineare Belastungscharakteristik z.B. an das Körpergewicht des Patienten angepasst werden. Darüberhinaus kann selbstverständlich sowohl durch die Wahl der Materialien, aus denen der Nucleus bzw. der Faserring aufgebaut sind, sowie durch die konkrete Gestaltung der Geometrie der die künstliche Bandscheibe aufbauenden Komponenten, das elastische Verhalten beeinflusst werden.

Die den Faserrings aufbauenden Materialien umfassen, soweit sie mit Körpergewebe in Berührung kommen, körperverträgliche Kunststoffe. Der Faserring selbst kann durchlässig für körpereigene Flüssigkeiten, muss jedoch vollständig undurchlässig für den Nucleus sein. Die Endplatten können aus einem Metall, beispielsweise aus zähfestem Titan oder aus einer zähfesten Titanlegierung gefertigt sein, welche für die Verankerung des Faserschlauches plastisch deformierbar ist. Die Aussenflächen der Endplatten können Zonen mit Metallgewebe aufweisen, die das Einwachsen von Knochenmaterial, und damit das Verwachsen der angrenzenden Wirbel mit den äusseren Flächen der Endplatten erleichtern. Eine Verbesserung der Verankerung zwischen Endplatte und Wirbel bezüglich Seitenkräften kann durch eine oder mehrere vorstehende Rippen erreicht werden, die z.B. von ventral nach dorsal verlaufen. Eine zusätzliche Zahnung am Aussenrand der Endplatte, die, ebenso wie die vorstehende Rippe, nicht zwingend vorhanden sein muss, kann ebenfalls die Verbindung zwischen Endplatte und Wirbel bezüglich Scherbeanspruchungen deutlich verbessern. Dabei muss die Endplatte nicht unbedingt aus Metall aufgebaut sein, sondern es können beispielsweise auch geeignete körperverträgliche Kunststoffe als Materialien für den Aufbau der Endplatte in Betracht kommen.

Im folgenden wird die Erfindung anhand von bevorzugten Ausführungsbeispielen, sowie der Zeichnung näher erläutert. Es zeigen:
- Fig. 1: Schematisch einen Längsschnitt durch eine erfindungsgemässe künstliche Bandscheibe mit parallel zueinander stehenden Endplatten;
- Fig. 2: eine weiteres Ausführungsbeispiel analog zu Fig. 1 mit Endplatten, die in einem spitzen Winkel zueinander stehen;
- Fig. 3: schematisch eine Ansicht einer weiteren künstlichen Bandscheibe;
- Fig. 4: eine erfindungsgemässe künstliche Bandscheibe eingesetzt zwischen zwei benachbarte Wirbelkörper;
- Fig. 5: schematisch ein Kraft - Weg Diagramm des elastischen Verhaltens einer erfindungsgemässen künstlichen Bandscheibe im Vergleich zu einer bekannten Elastomerprothese;
- Fig. 6: schematisch ein Ausführungsbeispiel zur Verbindung eines Faserrings mit einer Endplatte mittels eines ringförmigen Klemmelementes;
- Fig. 7: schematisch eine zweite Variante eines Ausführungsbeispiels gemäss Fig. 6;
- Fig. 8: schematisch eine dritte Variante eines Ausführungsbeispiels gemäss Fig. 6, wobei der Faserring zusätzlich verklebt oder verschweisst ist;
- Fig. 9: schematisch ein Ausführungsbeispiel zur Verbindung des Faserrings mit einer Endplatte, wobei der Faserring verklebt, verschweisst oder einen Kunststoff angespritzt ist;
- Fig. 10: schematisch ein Ausführungsbeispiel zur Verbindung des Faserrings mit einer Endplatte, wobei der Faserring durch ein Halteelement fixiert ist;
- Fig. 11: schematisch ein weiteres Ausführungsbeispiel gemäss Fig. 10;
- Fig. 12: schematisch ein Ausführungsbeispiel zur Verbindung des Faserrings mit einer Endplatte, wobei der Faserring in einer Nut kalt verpresst ist;
- Fig. 13: schematisch ein zweites Ausführungsbeispiel gemäss Fig. 12;
- Fig. 14: schematisch ein drittes Ausführungsbeispiel gemäss Fig. 12.

Die Erfindung wird im folgenden anhand bevorzugter Ausführungsbeispiele erläutert. Die erfindungsgemässe künstliche Bandscheibe B zum Implantieren zwischen zwei Wirbelkörper W, umfasst zwei Endplatten 1, 2, die einen vollständig mit einem elastisch und / oder plastisch verformbaren Nucleus 3 ausgefüllten Hohlraum 4 an zwei gegenüberliegenden Seiten begrenzen. Dabei wird der Hohlraum 4 von einem rohrförmigen Faserring 5 derart umschlossen, dass die Endplatten 1, 2 zusammen mit dem Faserring 5 den Hohlraum 4 begrenzen. Die Endplatten 1, 2 stehen mit dem Faserring 5 in zugfester Verbindung, wobei der Faserring 5 in radialer und axialer Richtung elastisch dehnbar ausgeführt ist und der Faserring 5 ein grösseres Elastizitätsmodul als der Nucleus 3 aufweist. Der Nucleus 3 und der Faserring 5 wirken derart zusammen, dass die elastischen Eigenschaften der künstlichen Bandscheibe mindestens in Bezug auf eine Kompressionskraft F mit zunehmender Verformung ein nicht-lineares Verhalten zeigen.

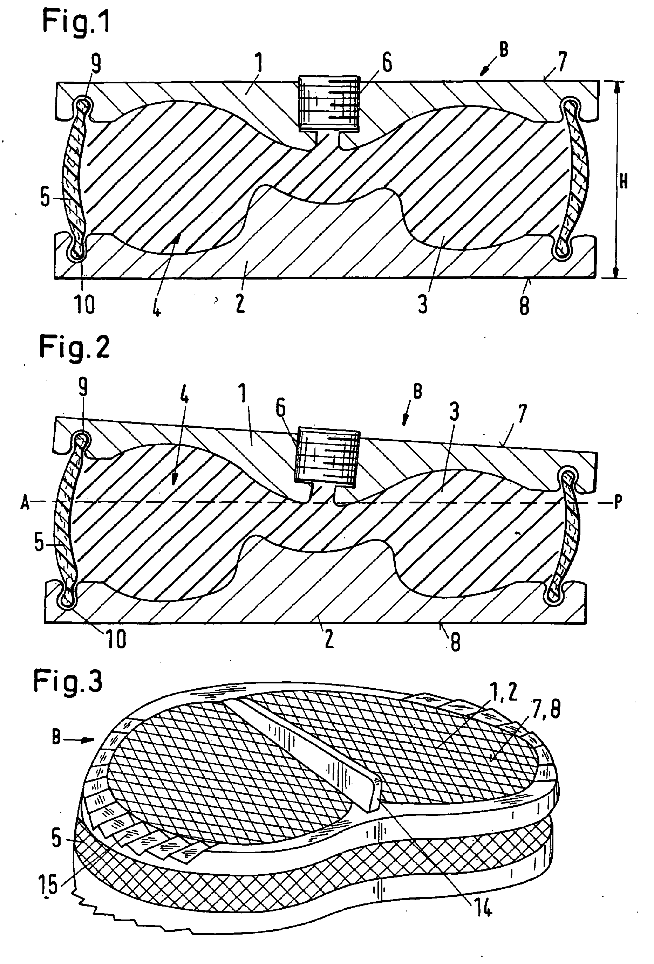

Fig. 1 zeigt in einer schematischen Darstellung ein bevorzugtes Ausführungsbeispiel einer erfindungsgemässen künstlichen Bandscheibe B. Zwischen den Endplatten 1, 2 ist ein Nucleus 3 mittels des Faserrings 5 verspannt. Die Endplatten 1,2 sind so ausgestaltet, dass eine möglichst grosse Auflagefläche gegenüber den Wirbelkörpern W vorliegt, um die Flächenpressung so klein wie möglich zu halten und ein Eindringen der Prothese in den Wirbelkörper zu verhindern. Die Bauhöhe H der künstlichen Bandscheibe kann entweder konstant sein, oder, wie in Fig. 2 gezeigt, z.B. von anterior A nach posterior P abnehmend sein. Dabei kann eine Variation der Bauhöhe H der künstlichen Bandscheibe B auch dadurch erreicht werden, dass der Faserring 5 eine über seinen gesamten Umfang konstante Höhe aufweist, die Dicke der Endplatten 1, 2 jedoch entsprechend variiert. Die Primärverankerung der künstlichen Bandscheibe B mit dem Wirbelkörper W erfolgt über eine geeignete Präparation der Endplatten 1, 2, so dass eine formschlüssige Verbindung zwischen Endplatte 1, 2 und Wirbelkörper W gewährleistet wird. Dieser Formschluss erfolgt gemäss Fig. 3 über eine Zahnung 15 und / oder eine vorstehende Rippe 14, wobei eine Verankerung vorwiegend im Bereich der kortikalen Randleisten erfolgt, da sich dort die höchsten Kräfte übertragen lassen. Die Sekundärverankerung der Wirbelkörper W an den Endplatten 1, 2 wird dadurch erreicht, dass die nach aussen gerichteten Flächen 7, 8 der Endplatten 1, 2 als Verankerungsplattformen ausgebildet sind, so dass ein dauerhaftes Verwachsen der angrenzenden Wirbelkörper W mit den nach aussen gerichteten Flächen 7, 8 der Endplatten 1, 2 ermöglicht wird. Konkret können die Verankerungsplattformen z.B. mit Gitterlagen aus Titan oder Titanlegierungen als "mesh" oder in Form anderer netzartiger oder poröser Strukturen aus Titan oder Titanlegierungen mit möglichst grossen Kontaktflächen ausgeführt sein, um ein dauerhaftes Einwachsen der Wirbelkörper W an der künstlichen Bandscheibe B zu gewährleisten. Die Endplatte 1, 2 selbst kann aus einem chirurgischen Stahl oder aus einem Kunststoff, wie z.B. einem Hochleistungspolymer wie Polyetheretherketon (PEEK), PEEK-Karbon oder Polyimid gefertigt sein. Bevorzugt werden Titan oder Titanlegierungen verwendet, die wegen ihrer hervorragenden Knochenverträglichkeit und des ausgezeichneten Einwachsverhaltens von Knochenmaterial an ihnen bekannt sind, und die - sofern sie gute Kalt-Umformeigenschaften besitzen - eine einfache und zuverlässige Verankerung des Faserrings 5 an den Endplatten 1, 2 gestatten. Zudem kann die Verbindung der Verankerungsplattform zu den Gitterlagen aus Titan oder Titanlegierungen durch Schweissung erfolgen.

Der Nucleus füllt bevorzugt den gesamten Hohlraum 4 aus, der von den Endplatten 1, 2 und dem Faserring 5 gebildet wird. Die Kernmaterialien, die für den Nucleus 3 in Betracht kommen, sollten im wesentlichen inkompressibel sein, einen Elastizitätsmodul besitzen, dessen Wert kleiner als 100 MPa ist und in der verwendeten Form körperverträglich sein. Als Kernmaterialien kommen für den Nucleus 3 z.B. Hydrogele, Kunststoffe auf Silikonbasis, Polycarbourethan (PCU) oder andere geeignete elastische oder plastische Kunstoffe oder Gummimaterialien in Frage. Das Kernmaterial, das den Nucleus bildet, wird bevorzugt flüssig in den Hohlraum 4 der künstlichen Bandscheibe eingebracht, die dazu eine verschliessbare Öffnung 6 in einer der Endplatten 1, 2 aufweist. Nach dem Einfüllen des Kernmaterials kann dieses sich je nach Material z.B. zu einem elastisch und / oder plastisch verformbaren Nucleus 3 verfestigen und z.B. durch Polymerisation oder auf andere Weise aushärten. Selbstverständlich ist auch denkbar, dass der Nucleus eine hohe plastische Verformbarkeit auf Dauer behält oder sogar eine mehr oder weniger flüssigkeitsartige Konsistenz beibehält. Die Befüllung des Hohlraumes 4 erfolgt bevorzugt nicht intraoperativ, was jedoch grundsätzlich ebenfalls möglich ist. Je nach gewünschter Charakteristik für das elastische Verhalten der künstlichen Bandscheibe B wird das Kernmaterial mit dem entsprechenden Druck eingefüllt und der Faserring 5 so unter eine gewisse Vorspannung gesetzt.

Der Faserring 5 bildet eines der technologischen Kernelemente der künstlichen Bandscheibe B. Er verleiht der künstlichen Bandscheibe B im Zusammenspiel mit dem Nucleus 3 die elastische Charakteristik für alle Belastungsarten. Der Faserring 5 besteht aus hochfesten Fasern mit hohem Elastizitätsmodul. Als bevorzugte Materialien kommen Stoffe wie Polyethylen Terephthalat (PET) aber auch Kunststoffe wie Polyamid, Polyimid, Kohlefasern oder z.B. Polyetheretherketon (PEEK) in Frage. Der Faserringe 5 wird dabei bevorzugt in einer oder mehreren Lagen geflochten, gewoben oder gestrickt oder auf andere Weise hergestellt, wobei die einzelnen Lagen gleiche oder unterschiedliche Flechtwinkel aufweisen können. Dabei sind ganz verschiedene Herstellungsverfahren denkbar. Beispielsweise können zunächst flache Bänder geflochten werden, die dann zu Ringen vernäht oder z.B. ultraschallgeschweist werden. Durch den Flechtvorgang kann eine Randzone des Faserrings 5 leicht dicker geflochten werden, wodurch der Faserring 5 besser in der Endplatte 1, 2, z.B. in einer Nut 9, 10, verankert werden kann. Eine weitere Variante besteht darin Schläuche zu flechten, die zu Ringen vernäht oder z.B. ultraschallgeschweisst werden können. In die Randzonen der Ringe können Fasern oder Bänder, z.B. aus Polyethylen Terephthalat (PET) oder Titan, einer Titanlegierung oder eines anderen geeigneten Materials eingelegt werden, wodurch eine Verdickung der Randzone erreicht wird, die zur Befestigung des Faserrings 5 an der Endplatte 1, 2, z.B. in der Nut 9, 10, geeignet ist. Eine weitere Variante zur Herstellung des Faserrings 5 besteht im Flechten eines Endlosschlauches mit einem für den Faserring 5 erforderlichen Durchmesser. Von diesem Schlauch, der selbstverständlich ebenfalls aus einer oder mehreren Lagen bestehen kann, wird der Faserring 5 z.B. thermisch, mittels Ultraschall oder auf andere Weise abgetrennt, wodurch beliebige Höhen für den Faserring 5 realisierbar sind. Beim Abtrennvorgang kann eine Randzone im Abtrennbereich thermisch so aufgeschmolzen und geformt werden, dass eine optimale Klemmgeometrie resultiert, so dass der Faserring 5 sicher in der Endplatte 1, 2, z.B. in der Nut 9, 10, verankert werden kann. Dabei muss in jedem Fall sichergestellt sein, dass es auch unter sehr hohen Drücken, wie sie auf die künstliche Bandscheibe B als Implantat in einer menschlichen Wirbelsäule einwirken können, nicht dazu kommt, dass der Nucleus 3 oder ein Teil davon, durch den Faserring 5 oder auf anderem Wege austreten kann. Daher sind hohe Anforderungen an die Herstellung des Faserrings 5 gestellt. Er muss unter anderem so dicht gearbeitet sein, dass der Nucleus 3 oder Teile davon den Faserring 5 nicht durchdringen können. Dazu kann der Faserring 5 z.B. zusätzlich eine dichtende Schicht aufweisen. Andererseits kann es von Vorteil sein, dass der Hohlraum 4 mit der Umgebung Körperflüssigkeit austauschen kann. Dies ist insbesondere von Vorteil, wenn als Materialien für den Nucleus 3 z.B. Hydrogele verwendet werden. Hydrogele haben die Eigenschaft, dass sie mit zunehmendem oder abnehmendem Hydrationsgrad starke korrespondierende Volumenänderungen aufweisen können. Dabei kann allgemein der Zusammenhang festgestellt werden, dass Hydrogele mit zunehmendem Hydrationsgrad ihr Volumen durch Aufquellen stark vergrössern können und in diesem Zustand einen verhältnismässig kleinen Elastizitätsmodul aufweisen und daher für Anwendungen ohne räumliche Begrenzung ungeeignet sind. Da bei der erfindungsgemässen künstlichen Bandscheibe B die räumliche Eingrenzung des Hohlraumes 4 durch den Faserring 5 und die Endplatten 1, 2 gewährleistet ist, können auch Hydrogele mit hohem Hydrationsgrad als Material für den Nucleus 3 in Betracht kommen. Den Effekt der Hydration des Hydrogels kann bei der erfindungsgemässen künstlichen Bandscheibe B besonders vorteilhaft ausgenutzt werden. Wenn der Faserring 5 so ausgeführt ist, dass er für den Nucleus 3 vollständig undurchlässig, für bestimmte Körperflüssigkeiten, wie z.B. Wasser, jedoch durchlässig ist, kann der Volumeneffekt, das heisst das Aufquellen des Nucleus 3, der aus einem Hydrogel besteht, durch Hydration ausgenutzt werden. Die künstliche Bandscheibe B kann dann nämlich bei stark reduzierter Einbringhöhe, was eine Implantation erheblich vereinfacht, in dehydriertem Zustand in die Wirbelsäule als Ersatz für eine entfernte natürliche Bandscheibe eingebracht werden und trotzdem später das gezeigte nicht-lineare Verhalten aufweisen. Körperflüssigkeiten wie Wasser, das durch den Faserring 5 eindringen kann, erhöht nach der Implantation durch zunehmende Hydration das Volumen des Nucleus 3, der schliesslich nach einer bestimmten Zeit einen vorgesehenen Enddruck bei einem vorbestimmten Endvolumen erreicht.

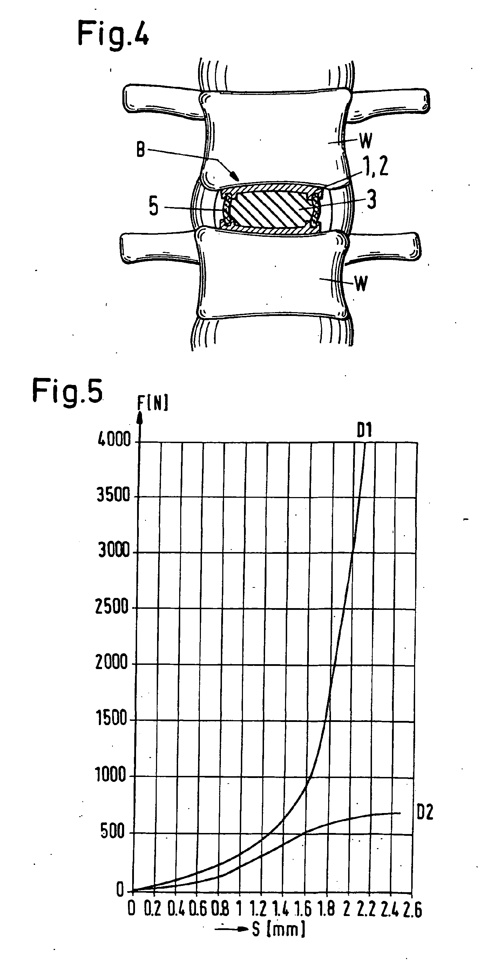

Entscheidend für das mechanische Verhalten der erfindungsgemässen künstlichen Bandscheibe B ist ihr stark nicht-lineares elastisches Verhalten z.B. unter einer Kompressionskraft F. Dabei beruht die Druckcharakteristik auf einem Zusammenspiel von Nucleus 3 mit dem Faserring 5. Fig. 5 zeigt exemplarisch ein Kraft - Weg Diagramm D1 des elastischen Verhaltens einer erfindungsgemässen künstlichen Bandscheibe B im Vergleich zu einem typischen Kraft - Weg Diagramm D2 einer bekannten Elastomerprothese, die keinen mit einem Nucleus mechanisch gekoppelten Faserring umfasst.

Wird die erfindungsgemässe künstliche Bandscheibe durch eine Kompressionskraft F, die z.B. senkrecht zu den Flächen 7, 8 der Endplatten 1, 2 wirkt, nur wenig, das heisst um einen Weg S von deutlich weniger als ungefähr 0.5 mm komprimiert, beeinflusst der Elastizitätsmodul des Nucleus 3 wesentlich die Belastungscharakteristik. In diesem Bereich verlaufen die Kraft - Weg Diagramme D1 und D2 noch im wesentlichen parallel. Mit zunehmendem Kompressionsweg S werden die Fasern des Faserrings 5 gestreckt und der Faserring 5 balgt aufgrund der Volumenverlagerung des Nucleus 3 leicht aus. Da der Nucleus 3 wenig kompressibel ist, transformiert dieser bei zunehmender Kompressionskraft F die auf die künstliche Bandscheibe B wirkenden Druckkräfte in den Faserring 5 und induziert in die Fasern des Faserrings 5 Zugkräfte. Damit bestimmt der Elastizitätsmodul des Faserrings 5 mit fortschreitender Kompression in zunehmendem Masse die Belastungscharakteristik, die, wie die Kurve D1 eindrucksvoll zeigt, stark progressiv anzusteigen beginnt. Die Kurve D2 demonstriert dagegen, dass das elastische Verhalten der bekannten Elastomerprothese kein progressiv ansteigendes Verhalten zeigt. Bei einem Kompressionsweg, der deutlich über 1 mm liegt zeigt das Kraft - Weg Diagramm D2 sogar ein degressives Verhalten, was auf ein unerwünschtes Ausbalgen des Elastomerkerns unter steigender Druckbelastung zurückzuführen ist. Bei der erfindungsgemässen künstlichen Bandscheibe B können dagegen die Fasern des den Nucleus 3 umschliessenden Faserrings 5 mit zunehmender Ausbalgung die auftretenden Kräfte immer besser aufnehmen und die daraus resultierende geometrische Übersetzung verstärkt die progressive Zunahme der Kraft-Weg Kurve D1 zusätzlich. Damit kann schon allein über den Aufbau des Faserrings die Belastungscharakteristik der künstlichen Bandscheibe B bedeutend beeinflusst werden.

Es ist ein besonderes Charaktersitikum des Kraft-Weg Diagramms in Fig. 5, dass, analog zum Verhalten einer natürlichen Bandscheibe, bereits die Anfangssteigung der Kurve leicht progressives Verhalten zeigt. Das heisst, die erfindungsgemässe künstliche Bandscheibe B zeigt bereits bei beliebig kleiner Kompressionskraft F und damit bei beliebig kleinen Belastungen, einen gewissen Anstieg der Anfangssteifigkeit, die von Null verschieden ist. Wobei unter Steifigkeit (bzw. Anfangssteifigkeit) der künstlichen Bandscheibe bei einer Kompressionskraft F die entsprechende Steigung der Kurve D1 bzw. D2 im Kraft-Weg Diagramm bei einem Deformationsweg S zu verstehen ist, der der entsprechenden Kompressionskraft F zugeordnet ist. So zeigt die erfindungsgemässe Bandscheibe unter einer Kompressionskraft F von 3000 N eine bevorzugte Steifigkeit zwischen 2000 N/mm und 20000 N/mm und unter einer Deformation S von ca. 0.4 mm eine Steifigkeit, deren Wert kleiner als 500 N/mm ist.

Da die nicht-linearen Eigenschaften der künstlichen Bandscheibe B, die diese bezüglich einer Kompressionskraft F aufweist, durch das Zusammenspiel von Faserring 5 und Nucleus 3 auch in das elastische Verhalten in Bezug auf Torsions- Biege- und Schubbelastungen transformiert werden, zeigt die künstliche Bandscheibe B auch bei entsprechenden beliebig kleinen Auslenkungen aus dem unbelasteten Zustand, gegenüber diesen Belastungsarten sowohl einen bestimmten Anstieg der Anfangssteifigkeit, als auch einen weiteren progressiven Anstieg, der der entsprechenden Belastungsart zugeordneten Steifigkeit. Da der erwähnte Anstieg der Anfangssteifigkeit dadurch erreicht wird, dass der Nucleus 3 im unbelasteten Zustand über den Faserring 5 unter einer gewissen Druckvorspannung steht, ergibt sich die Möglichkeit, unter Einhaltung einer vorgegebenen Geometrie für die künstlichen Bandscheibe B und ohne Veränderung oder Austausch der die künstliche Bandscheibe B aufbauenden Materialien, die elastischen Eigenschaften an die individuellen Bedürfnisse eines Patienten anzupassen. So kann die nicht-lineare Belastungscharakteristik und damit die Steifigkeit und deren Veränderung mit zunehmender Kompressionskraft F einfach durch eine geeignete Wahl der Druckvorspannung z.B. an das Körpergewicht des Patienten angepasst werden.

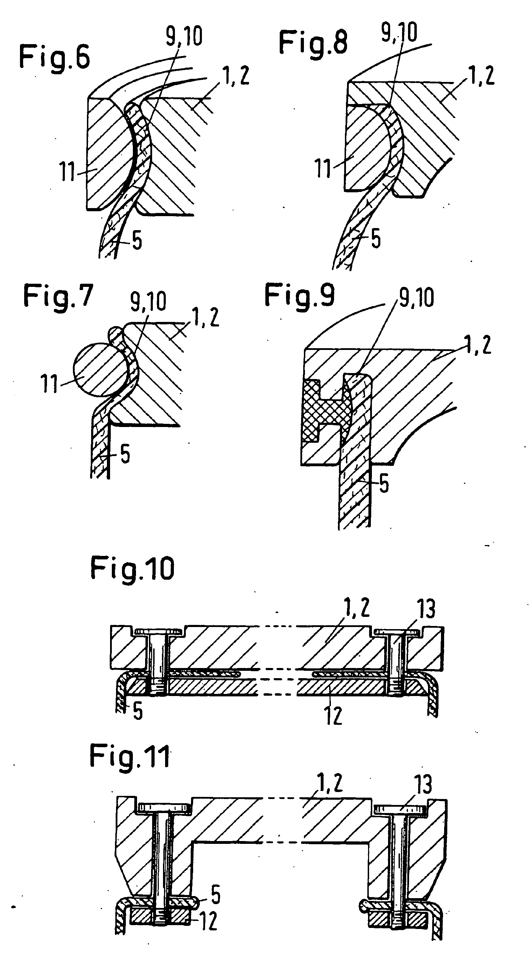

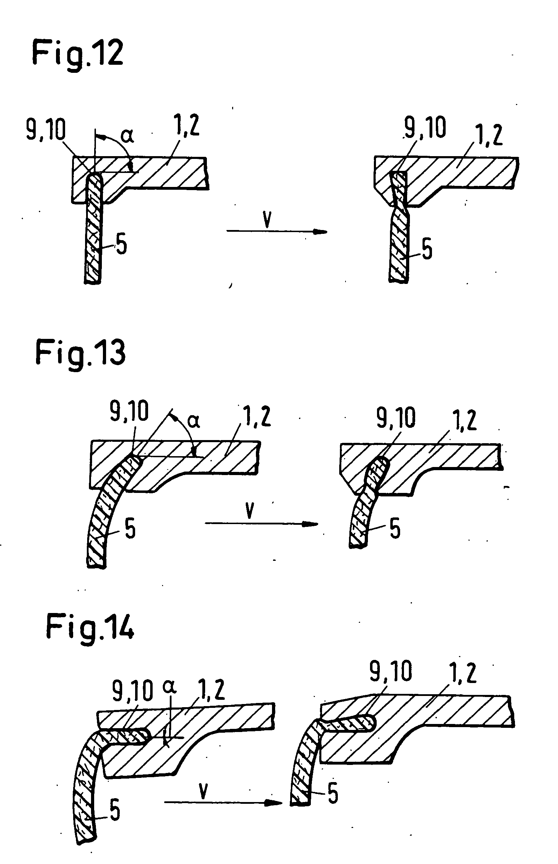

Voraussetzung für eine gute Funktion der erfindungsgemässen künstlichen Bandscheibe B ist eine einwandfreie Verankerung des Faserrings 5 in der Endplatte 1, 2. In den Fig. 6 bis 9 und den Fig. 12 bis 14 ist schematisch eine Nut 9, 10 in der Endplatte 1, 2 gezeigt, in der der Faserring 5 befestigt ist. Die Art der Befestigung hängt unter anderem vom Material der Endplatte 1, 2 ab. So kann der Faserring 5, wie in den Fig. 6, 7 gezeigt, an der Endplatte 1, 2, die aus Metall oder Kunststoff gefertigt ist, z.B. durch ein ringförmiges Klemmelement 11 in der Nut 9, 10 fixiert werden oder zusätzlich (siehe Fig. 8, 9) in der Nut 9, 10 verklebt sein. Bei der Endplatte 1, 2 aus Kunststoff ist alternativ zum Kleben auch thermisches Verschweissen in der Nut 9, 10 oder auf einer Oberfläche der Endplatte 1 ,2 möglich. Bei einer metallischen Endplatte 1, 2 kann der Faserring 5 durch Kaltumformung V in der Nut 9, 10 verpresst werden. Dabei kann die Nut 9, 10 wie in den Fig. 12 bis 14 gezeigt unter einem bestimmten Winkel α gegen die Oberfläche der Endplatte 1, 2 geneigt sein. Dabei wird ein Winkel α gewählt, der zwischen 0 Grad (Fig. 14) und 90 Grad (Fig. 12), vorzugsweise jedoch in der Nähe von 60 Grad liegt. Als weitere Variante für die Befestigung des Faserrings 5 an der Endplatte 1, 2 ist auch eine Fixierung mittels eines zusätzlichen Halteelementes 12 denkbar, das zum Beispiel als Platte (Fig. 10) oder als Ring (Fig. 11) ausgebildet sein kann und vorzugsweise mittels einer Verschraubung 13 an der Endplatte 1, 2 befestigt ist.

Durch die Erfindung wird eine künstliche Bandscheibe B vorgeschlagen, die sich aus mehreren Gründen besonders gut als Implantat und damit als Ersatz für eine natürliche Bandscheibe eignet. In ihrer Ausführung als einteiliges Implantat, das nur aus wenigen Komponenten zusammengesetzt ist, kann die erfindungsgemässe künstliche Bandscheibe B auf einfache Weise in die menschliche Wirbelsäule eingebracht werden. Sie kann bereits vorgefertigt und sterilisiert für einen entsprechenden operativen Eingriff angeliefert werden, so dass aufwendige Techniken zum Zusammensetzen von Einzelteilen während eines operativen Eingriffs innerhalb des menschlichen Körpers bzw. zwischen zwei Wirbelkörpern W, überflüssig werden. Die Distraktion der Wirbelkörper W zum Einbringen einer Bandscheibenprothese ist eine Aufgabe, die aufgrund der beengten Platzverhältnisse grundsätzlich ein erhebliches Problem darstellt. Wenn als Kernmaterial für den Nucleus 3 z.B. ein dehydriertes Hydrogel verwendet wird, weist die künstliche Bandscheibe B zum Zeitpunkt des Einbringens in die Wirbelsäule im dehydrierten Zustand eine um bis zu 50 % geringere Bauhöhe H auf, als im vollständig hydrierten Zustand. Die Probleme im Zusammenhang mit der Distraktion werden dadurch erheblich reduziert. Bedingt durch den besonderen Aufbau des Faserrings 5, das Zusammenwirken von Nucleus 3 und Faserring 5 und eine geeignete Wahl der Materialien, erhält das elastische Verhalten der erfindungsgemässen künstlichen Bandscheibe B in Bezug auf alle relevanten Belastungsarten eine typische nicht-lineare Charakteristik, das der einer natürlichen Bandscheibe nahezu identisch nachbildet ist. Konstruktionsbedingt wirkt dabei der Nucleus 3 als Krafttransformator, der die auftretenden Belastungen in den Faserring 5 transformiert, wodurch die künstliche Bandscheibe B eine naturgetreue elastische Charakteristik erhält.

## Patentansprüche

1. Künstliche Bandscheibe, zum Implantieren zwischen zwei Wirbelkörper (W), umfassend zwei Endplatten (1, 2), die einen mit einem elastisch und / oder plastisch verformbaren Nucleus (3) ausgefüllten Hohlraum (4) an zwei gegenüberliegenden Seiten begrenzen, wobei der Hohlraum (4) von einem rohrförmigen Faserring (5) derart umschlossen ist, dass die Endplatten (1, 2) zusammen mit dem Faserring (5) den Hohlraum (4) begrenzen und die Endplatten (1, 2) mit dem Faserring (5) in zugfester Verbindung stehen, **dadurch gekennzeichnet, dass** der Faserring (5) in radialer und axialer Richtung elastisch dehnbar ausgeführt ist, wobei der Faserring (5) ein grösseres Elastizitätsmodul als der Nucleus (3) aufweist und der Nucleus (3) und der Faserring (5) derart zusammenwirken, dass die elastischen Eigenschaften der künstlichen Bandscheibe mindestens in Bezug auf eine Kompressionskraft (F) mit zunehmender Verformung ein nicht-lineares Verhalten zeigen.

2. Künstliche Bandscheibe nach Anspruch 1, bei welcher der Nucleus (3) aus einem Material aufgebaut ist, dessen Elastizitätsmodul einen Wert kleiner als 100 MPa hat.

3. Künstliche Bandscheibe nach Anspruch 1 oder 2, bei welcher der Wert für die Steifigkeit der künstlichen Bandscheibe unter einer Kompressionskraft (F) von 3000 N in einem Bereich zwischen 2000 N/mm und 20000 N/mm liegt.

4. Künstliche Bandscheibe nach einem der Ansprüche 1 bis 3, bei welcher der Faserring (5) aus einem Gewebe, Gewirk oder Geflecht besteht und eine oder mehrere Lagen umfasst.

5. Künstliche Bandscheibe nach einem der Ansprüche 1 bis 4, bei welcher die den Faserring (5) aufbauenden Materialien einen oder mehrere Kunststoffe, wie z.B. Polyamid, Polyimid, Kohlefasern oder Polyetheretherketon (PEEK), vorzugsweise Polyethylen Terephthalat (PET), umfassen.

6. Künstliche Bandscheibe nach einem der Ansprüche 1 bis 5, bei welcher der Nucleus (3) aus einem Hydrogel und / oder einem elastischen Kunststoff auf Silikonbasis und / oder aus einem anderen elastischen Kunststoff aufgebaut ist.

7. Künstliche Bandscheibe nach einem der Ansprüche 1 bis 6, bei welcher der Hohlraum (4) durch eine verschliessbare Öffnung (6) in einer Endplatte (1,2) zugänglich ist.

8. Künstliche Bandscheibe nach einem der Ansprüche 1 bis 7, bei welcher die Bauhöhe (H) entweder konstant ist oder vorzugsweise von anterior (A) nach posterior (P) abnimmt.

9. Künstliche Bandscheibe nach einem der Ansprüche 1 bis 8, bei welcher die Endplatten (1, 2) aus einem Metall, vorzugsweise aus Titan, einer Titanlegierung oder aus Kunststoff bestehen.

10. Künstliche Bandscheibe nach einem der Ansprüche 1 bis 9, bei welcher die Endplatten (1, 2) als Verankerungsplattformen ausgebildet sind, um ein Verwachsen der angrenzenden Wirbelkörper (W) mit den nach aussen gerichteten Flächen (7, 8) der Endplatten (1, 2) zu erreichen.

11. Künstliche Bandscheibe nach einem der Ansprüche 1 bis 10, bei welcher eine der Endplatten (1, 2) eine in Umfangsrichtung verlaufende Nut (9, 10) zur Aufnahme des Faserrings (5) aufweist.

12. Künstliche Bandscheibe nach Anspruch 11, an welcher der Faserring (5) zur Verbindung mit einer der Endplatten (1,2) durch Kaltumformung in einer der Nuten (9, 10) verpresst ist.

13. Künstliche Bandscheibe nach Anspruch 11 oder 12, an welcher der Faserring (5) zur Verbindung mit einer der Endplatten (1, 2) durch ein ringförmiges Klemmelement (11) in einer der Nuten (9, 10) fixiert ist.

14. Künstliche Bandscheibe nach einem der Ansprüche 1 bis 13, an welcher der Faserring (5) zur Verbindung mit einer der Endplatten (1, 2) durch ein Halteelement (12), vorzugsweise mittels einer Verschraubung (13), fixiert ist.

15. Künstliche Bandscheibe nach einem der Ansprüche 10 bis 14, an welcher der Faserring (5) zur Verbindung mit einer der Endplatten (1, 2) in einer der Nuten (9,10) verklebt oder verschweisst ist.

16. Künstliche Bandscheibe nach einem der Ansprüche 11 bis 15, an welcher der Faserring (5) zur Verbindung mit einer der Endplatten (1, 2) ringförmig in einer der Nuten (9, 10) der Endplatten (1, 2) mit Kunststoff angespritzt ist.

17. Künstliche Bandscheibe nach einem der Ansprüche 1 bis 16, an welcher der Faserring (5) zur Verbindung mit einer der Endplatten (1, 2) auf einer der Oberflächen (7, 8) mit Kunststoff angespritzt ist.

18. Künstliche Bandscheibe nach einem der Ansprüche 1 bis 17, bei welcher die Endplatten (1,2) eine vorstehende mittlere Rippe (14) aufweisen, die z.B. von ventral nach dorsal verläuft.

19. Künstliche Bandscheibe nach einem der Ansprüche 1 bis 18, bei welcher die Endplatten (1,2) an ihrem Aussenrand eine zum Wirbel (W) hin gerichtete Zahnung (15) aufweisen, so dass durch ein Einwachsen von Knochenmaterial eine formschlüssige Verbindung für die Übertragung von Torsions- und Flexionskräften erreicht wird.

## Claims

1. An artificial intervertebral disc for implantation between two vertebral bodies (W) comprising two end plates (1, 2) which bound a hollow space (4) at two opposite sides, the hollow space (4) filled with an elastically and/or plastically deformable nucleus (3) and being enclosed by a tubular fibre ring (5) such that the end plates (1, 2), together with the fibre ring (5), bound the hollow space (4) and the end plates (1,2) are in connection resistant to tension with the fibre ring (5), **characterised in that** the fibre ring (5) is elastically extensible in the radial and axial directions, with the fibre ring (5) having a larger modulus of elasticity than the nucleus (3) and the nucleus (3) and the fibre ring (5) interacting such that the elastic properties of the artificial intervertebral disc show a non-linear behaviour with increasing deformation at least with respect to a compression force (F).

2. An artificial intervertebral disc in accordance with claim 1, in which the nucleus (3) is made up of a material whose modulus of elasticity has a value less than 100 MPa.

3. An artificial intervertebral disc in accordance with claim 1 or claim 2, in which the value for the stiffness of the artificial intervertebral disc lies below a compression force (F) of 3000 N in a range between 2000 N/mm and 20,000 N/mm.

4. An artificial intervertebral disc in accordance with any one of the claims 1 to 3, in which the fibre ring (5) consists of a fabric, knitted fabric or braided fabric and comprises one or more layers.

5. An artificial intervertebral disc in accordance with any one of the claims 1 to 4, in which the materials making up the fibre ring (5) comprise one or more plastics such as polyamide, polyimide, carbon fibres or polyether etherketone (PEEK), preferably polyethylene terephthalate (PET).

6. An artificial intervertebral disc in accordance with any one of the claims 1 to 5, in which the nucleus (3) is made up of a hydrogel and/or of an elastic plastic based on silicone and/or of another elastic plastic.

7. An artificial intervertebral disc in accordance with any one of the claims 1 to 6, in which the hollow space (4) is accessible through a closable opening (6) in an end plate (1, 2).

8. An artificial intervertebral disc in accordance with any one of the claims 1 to 7, in which the construction height (H) is either constant or preferably reduces from anterior (A) to posterior (P).

9. An artificial intervertebral disc in accordance with any one of the claims 1 to 8, in which the end plates (1, 2) consist of a metal, preferably of titanium, of a titanium alloy or of plastic.

10. An artificial intervertebral disc in accordance with any one of the claims 1 to 9, in which the end plates (1, 2) are made as anchoring platforms in order to achieve an intergrowth of the adjoining vertebral bodies (W) with the outwardly directed surfaces (7, 8) of the end plates (1, 2).

11. An artificial intervertebral disc in accordance with any one of the claims 1 to 10, in which one of the end plates (1, 2) has a groove (9, 10) extending in the peripheral direction in order to receive the fibre ring (5).

12. An artificial intervertebral disc in accordance with claim 11 or claim 12, at which the fibre ring (5) is pressed in one of the grooves (9, 10) by cold shaping for connection to one of the end plates (1, 2).

13. An artificial intervertebral disc in accordance with claim 11, at which the fibre ring (5) is fixed in one of the grooves (9, 10) by an annular clamping element (11) for connection to one of the end plates (1, 2).

14. An artificial intervertebral disc in accordance with any one of the claims 1 to 13, at which the fibre ring (5) is fixed by a holding element (12), preferably by means of a screw connection (13), for connection to one of the end plates (1, 2).

15. An artificial intervertebral disc in accordance with any one of the claims 10 to 14, at which the fibre ring (5) is adhesively bonded or welded to one of the grooves (9, 10) for connection to one of the end plates (1, 2).

16. An artificial intervertebral disc in accordance with any one of the claims 11 to 15, at which the fibre ring (5) is injection moulded with plastic in an annular manner in one of the grooves (9, 10) of the end plates (1, 2) for connection to one of the end plates (1, 2).

17. An artificial intervertebral disc in accordance with any one of the claims 1 to 16, at which the fibre ring (5) is injection moulded with plastic at one of the surfaces (7, 8) for connection to one of the end plates (1, 2).

18. An artificial intervertebral disc in accordance with any one of the claims 1 to 17, in which the end plates (1, 2) have a projecting central rib (14) which extends, for example, from ventral to dorsal.

19. An artificial intervertebral disc in accordance with any one of the claims 1 to 18, in which the end plates (1,2) have a toothed arrangement (15) directed towards the vertebral body (W) at their outer edge so that a shape-locked connection is achieved for the transfer of torsion and flexion forces by an intergrowth of bone material.

## Revendications

1. Disque intervertébral artificiel, destiné à être implanté entre deux vertèbres (W), comprenant deux plaques terminales (1, 2) qui délimitent sur deux côtés opposés une cavité (4) remplie d'un noyau (3) élastiquement et/ou plastiquement déformable, ladite cavité (4) étant enfermée par une bague fibreuse (5) de forme tubulaire de telle façon que les plaques terminales (1, 2) délimitent la cavité (4) conjointement avec la bague fibreuse (5), et les plaques terminales (1, 2) sont avec la bague fibreuse (5) dans une liaison résistante à la traction, **caractérisé en ce que** la bague fibreuse (5) est réalisée de façon à pouvoir se dilater élastiquement en direction radiale et en direction axiale, ladite bague fibreuse (5) ayant un module d'élasticité plus élevé que le noyau (3), le noyau (3) et la bague fibreuse (5) coopérant de telle façon que les propriétés élastiques du disque intervertébral artificiel présentent un comportement non linéaire au fur et à mesure que la déformation progresse, au moins par référence à une force de compression (F).

2. Disque intervertébral artificiel selon la revendication 1, dans lequel le noyau (3) est réalisé en un matériau dont le module d'élasticité a une valeur inférieure à 100 MPa.

3. Disque intervertébral artificiel selon la revendication 1 ou 2, dans lequel la valeur pour la raideur du disque intervertébral artificiel sous une force de compression (F) de 3000 N est dans une plage entre 2000 N/mm et 20 000 N/mm.

4. Disque intervertébral artificiel selon l'une des revendications 1 à 3, dans lequel la bague fibreuse (5) est constituée par un tissu, un textile ou un treillis et comprend une ou plusieurs couches.

5. Disque intervertébral artificiel selon l'une des revendications 1 à 4, dans lequel les matériaux constituant la bague fibreuse (5) comprennent une ou plusieurs matières plastiques, comme par exemple polyamide, polyimide, fibre de carbone ou polyétheréthercétone (PEEC), de préférence polyéthylène téréphtalate (PET).

6. Disque intervertébral artificiel selon l'une des revendications 1 à 5, dans lequel le noyau (3) est réalisé à partir d'un hydrogel et/ou d'une matière plastique élastique à base de silicone et/ou d'une autre matière plastique élastique.

7. Disque intervertébral artificiel selon l'une des revendications 1 à 6, dans lequel la cavité (4) est accessible via une ouverture (6) refermable dans une plaque terminale (1, 2).

8. Disque intervertébral artificiel selon l'une des revendications 1 à 7, dans lequel la hauteur structurelle (H) est constante ou diminue de préférence du côté antérieur (A) vers le côté postérieur (P).

9. Disque intervertébral artificiel selon l'une des revendications 1 à 8, dans lequel les plaques terminales (1, 2) sont en un métal, de préférence en titane, en alliage de titane, ou en matière plastique.

10. Disque intervertébral artificiel selon l'une des revendications 1 à 9, dans lequel les plaques terminales (1, 2) sont réalisées comme des plates-formes d'ancrage, pour atteindre une croissance des vertèbres adjacentes (W) avec les surfaces (7, 8) dirigées vers l'extérieur des plaques terminales (1, 2).

11. Disque intervertébral artificiel selon l'une des revendications 1 à 10, dans lequel l'une des plaques terminales (1, 2) comporte une gorge (9, 10) s'étendant en direction périphérique pour recevoir la bague fibreuse (5).

12. Disque intervertébral artificiel selon la revendication 11, dans lequel la bague fibreuse (5) est pressée par déformation à froid dans l'une des gorges (9, 10) pour la liaison avec l'une des plaques terminales (1, 2).

13. Disque intervertébral artificiel selon la revendication 11 ou 12, dans lequel la bague fibreuse (5) est fixée dans l'une des gorges (9, 10) par un élément de coincement (11) de forme annulaire pour la liaison avec l'une des plaques terminales (1,2).

14. Disque intervertébral artificiel selon l'une des revendications 1 à 13, dans lequel la bague fibreuse (5) est fixée par un élément de maintien (12), de préférence au moyen d'un vissage (13) pour la liaison avec l'une des plaques terminales (1, 2).

15. Disque intervertébral artificiel selon l'une des revendications 10 à 14, dans lequel la bague fibreuse (5) est collée ou soudée dans l'une des gorges (9, 10) pour la liaison avec l'une des plaques terminales (1, 2).

16. Disque intervertébral artificiel selon l'une des revendications 11 à 15, dans lequel la bague fibreuse (5) est surmoulée de matière plastique sous forme annulaire dans l'une des gorges (9, 10) des plaques terminales (1, 2) pour la liaison avec l'une des plaques terminales (1,2).

17. Disque intervertébral artificiel selon l'une des revendications 1 à 16, dans lequel la bague fibreuse (5) est surmoulée avec une matière plastique sur l'une des surfaces (7, 8) pour la liaison avec l'une des plaques terminales (1, 2).

18. Disque intervertébral artificiel selon l'une des revendications 1 à 17, dans lequel les plaques terminales (1, 2) présentent une nervure médiane (14) en saillie, qui s'étend par exemple de la direction ventrale vers la direction dorsale.

19. Disque intervertébral artificiel selon l'une des revendications 1 à 18, dans lequel les plaques terminales (1, 2) comportent à leur bordure extérieure une denture (15) dirigée vers la vertèbre (W), de sorte que l'on obtient une liaison à coopération de formes grâce à une croissance du matériau osseux, pour la transmission de forces de torsion et de forces de flexion.
